# FASCICULE DE BREVET EUROPEEN

(11) **EP 1 476 422 B1**
(45) Date de publication et mention de la délivrance du brevet: **30.05.2007**
(21) Numéro de dépôt: 03718875.2
(22) Date de dépôt: 14.02.2003
(51) Int. Cl.: C07C 303/44, C07C 311/48

(54) **PROCEDE DE TRAITEMENT D'UNE SOLUTION ORGANIQUE D'IMIDE PORTEUR DE GROUPE SULFONYLE**
VERFAHREN ZUR BEHANDLUNG EINER ORGANISCHEN LÖSUNG EINES SULFONYLGRUPPE TRAGENDEN IMIDS
METHOD FOR TREATING AN IMIDE ORGANIC SOLUTION BEARING A SULPHONYL GROUP

(30) Priorité: 20.02.2002 FR 0202137
(43) Date de publication de la demande: 17.11.2004
(73) Titulaire: RHODIA CHIMIE, 92512 Boulogne Billancourt Cedex (FR)
(72) Inventeur: BESSON, Bernard, F-01700 Les Echets (FR)
(74) Mandataire: Dutruc-Rosset, Marie-Claude
(86) Numéro de dépôt international: PCT/FR2003/000482
(87) Numéro de publication internationale: WO 2003/070694

(56) Documents cités:
- EP-A- 0 364 340
- H. MATSUMOTO, ET AL.: "Highly conductive room temperature molten salts based on small trimethylalkylammonium cations and bis(trifluoromethylsulphonyl)imide" CHEMISTRY LETTERS, no. 8, août 2000 (2000-08), pages 922-923, XP002219330 Chemical Society of Japan, Tokyo, JP ISSN: 0366-7022
- P. WASSERSCHEID, ET AL.: "Synthesis and properties of ionic liquids derived from the 'chiral pool'" CHEMICAL COMMUNICATIONS, no. 3, 7 janvier 2002 (2002-01-07), pages 200-201, XP002219331 Royal Society of Chemistry, Letchworth, GB ISSN: 1359-7345

## Description

La présente invention a pour objet une technique de purification des imides sulfonés à partir de mélanges impurs en contenant. Elle vise plus particulièrement la purification des imides sulfonés dont le soufre porteur de la fonction sulfonique est rattaché à un atome perhalogéné, avantageusement perfluoré. Les imides porteurs d'une fonction sulfonique, elle-même reliée à un atome perhalogéné, bien qu'ils soient connus depuis des lustres, se développent actuellement rapidement. La raison en est que ces composés sont utilisés comme éléments constitutifs des sels de lithium se trouvant dans des batteries à performance élevée.

Il convient de rappeler que les imides sont des composés présentant la fonction ci-après :

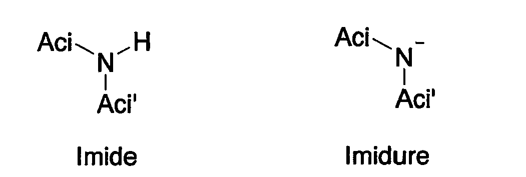

La fonction imidure n'est que la forme anionique déprotonée de l'imide qui présente deux substituants qui sont des restes correspondant à des acides oxygénés dont on a éliminé un groupement OH. Les imides, ou imidures, visés par la présente invention sont ceux dont l'un des Aci et Aci' est un groupement sulfonyle, avantageusement les deux Aci et Aci' sont sulfonyles. La fonction sulfonyle, ou l'une des fonctions sulfonyles quand il y en a deux, est portée par un carbone perhalogéné, avantageusement perfluoré. Sont plus particulièrement visés, les imides comportant un carbone perhalogéné sur chacun des substituants (ici Aci et Aci'), ledit carbone perhalogéné étant avantageusement porté par un atome lié directement à l'azote.

Ces imidures ont notamment des propriétés qui les ont désignés comme étant des matériaux de valeur pour la fabrication de piles ou de batteries, propriétés qui sont, d'une part, une acidité très élevée de l'imide et, d'autre part, une absence de pouvoir complexant de l'imidure vis-à-vis des différents cations.

Ces mêmes propriétés rendent extrêmement difficiles et délicates les opérations de purification. La forte acidité implique une forte dissociation et une forte dissociation implique une solubilité élevée dans les milieux polaires et notamment dans l'eau. En outre, ces composés très acides forment, en général, des composés d'addition avec l'eau et il est souvent très difficile de séparer ces composés très acides avec l'eau. Les techniques les plus usuelles pour purifier ces imides sont des techniques de recristallisation, notamment de recristallisation de leurs sels.

Ces imides sont, en général, synthétisés par l'action d'halogénures de sulfonyle sur des dérivés azotés trivalents porteurs d'hydrogène (tels que et notamment ammoniac et amide) et, dans des cas difficiles, porteurs de groupement trialcoylsilyle. Le groupe sulfonyle est, en général, porteur d'un carbone perhalogéné relié directement au soufre.

Les halogénures de sulfonyle les plus utilisés actuellement sont les fluorures et les chlorures de perfluoroalcanesulfonyle et notamment les fluorures et les chlorures de perfluoroalcanesulfonyle en C₁ et en C₂.

Des techniques de synthèse de ce type de composés sont décrites notamment dans des demandes de brevets de la société Central Glass et de la Demanderesse.

La difficulté de ce type de synthèse conduit à la présence d'impuretés en quantités significatives et notamment des acides sulfoniques et sulfiniques correspondant au chlorure d'acide de sulfonyle. On trouve également comme impuretés, le dérivé azoté de départ ainsi que les amines qui sont souvent utilisées pour neutraliser les acides formés au cours de la réaction, et les éventuelles amines d'un type particulier qui sont utilisées comme catalyseurs.

Ainsi, H. Matsumuto et al, Chemistry Letters 2000, (8), 922-923 décrivent un procédé de préparation de bis(trifluorométhyl)sulfoimidures de tétraalkylammonium et le produit obtenu est soumis à une opération de lavage jusqu'il n'y ait plus d'atome d'halogène résiduel.

Une autre source de ces imides ou de leurs sels, les imidures, est le recyclage de catalyseurs ou de piles usagées. On retrouve dans ces produits de recyclage, le même type d'impuretés que celles qui viennent d'être décrites ci-dessus, sauf peut-être celles qui dérivent des amines utilisées comme catalyseurs (telles que et notamment les dialcoylaminopyridines), les autres amines pouvant être introduites pour faciliter la séparation et la récupération des constituants des piles.

Par ailleurs les techniques d'extraction liquide/liquide ne sont en général guère purifiantes, l'ensemble des composés organiques sont extraits dans la phase organique ; en outre, les composés synthétisés et/ou les impuretés ainsi que les composés de départ sont amphiphiles, jouent le rôle de tiers-solvant et sont même susceptibles de présenter des propriétés tensioactives. Les amines grasses sont, en outre, connues pour être amphiphile.

En outre, pour pouvoir réaliser dans de bonnes conditions des purifications par extraction (liquide-liquide), il faut, d'une part, que les coefficients de partage entre, d'un côté, le(s) produit(s) à garder et, de l'autre côté, des impuretés à éliminer soient très différents et, d'autre part, que le coefficient de partage de l'impureté à éliminer ne soit pas trop faible vis-à-vis de la phase vecteur de ladite impureté.

En d'autres termes, si la phase organique φₒ est celle d'où l'on désire retirer le ou les composés considérés comme impureté(s) et garder les composés convoités et la phase aqueuse φₐ, la phase lavante, alors pour éviter d'avoir trop de flux à manipuler il convient que le coefficient de partage de l'impureté (φₐ/φₒ) considérée, à savoir le rapport à l'équilibre entre la teneur en impureté par unité de quantité de matière dans la phase aqueuse φₐ et la teneur en impureté par unité de quantité de matière dans la phase organique φₒ, soit aussi élevé que possible et en tout cas au moins égal à 1, avantageusement à 2, de préférence à 5. Bien sûr, lorsque la phase lavante est la phase organique φₒ, la condition à observer est la réciproque, à savoir que c'est le coefficient de partage (φₒ/φₐ) qui doit être élevé et au moins égal à 1, avantageusement à 2, de préférence à 5.

Il convient enfin de souligner que le caractère amphiphile des acides à carbone(s) polyfluoré(s) rend très difficile la prévision de leur comportement.

C'est pourquoi, un des buts de la présente invention est de fournir un procédé qui permette la récupération et l'enrichissement des solutions contenant les imides ou les imidures visés ci-dessus.

Un autre but de la présente invention est de fournir un procédé du type ci-dessus qui permette de séparer les imides, ou les imidures, d'avec les acides sulfoniques correspondant au sulfonyle constitutif d'au moins une des branches de la fonction imide ou imidure.

Un autre but de la présente invention est de fournir un procédé du type ci-dessus qui permette de séparer les imides, ou les imidures, d'avec les acides, leurs sels sulfiniques correspondant à la réduction des groupes sulfonyles constitutifs d'une des branches de la fonction imide.

Un autre but de la présente invention est de fournir une technique qui permette de séparer les imides, ou les imidures, d'avec les différents halogénures susceptibles d'avoir été dégagés, d'être présents dans le mélange réactionnel ou de recyclage. Les halogénures les plus couramment dégagés sont les ions fluorures et/ou les ions chlorure.

Ces buts, et d'autres qui apparaîtront par la suite, sont atteints au moyen d'un procédé de traitement d'une composition organique impure, d'imidure d'ammonium, imidure dont l'un des substituants, avantageusement les deux, est un sulfonyle porté par un carbone perhalogéné, de préférence perfluoré, caractérisé par le fait que l'on soumet ladite composition à une étape d'extraction de liquide-liquide au moyen d'une phase aqueuse Φₐ présentant un pH ajusté à une valeur au moins égale 9 et par le fait que ladite composition organique impure comporte comme impuretés au moins l'une des espèces chimiques choisies parmi les halogénures, les sulfinates et les sulfonates.

Il est opportun de mentionner que les essais antérieurs dissuadaient l'homme du métier d'utiliser de telles techniques pour réaliser une purification poussée. En effet, les coefficients de partage obtenus à partir de mélange réactionnel étaient très peu favorables. Une explication plausible est qu'il y avait trop de composés organiques, notamment de composés basiques (salifiés ou non, surtout sous forme d'halohydrate), dans la phase aqueuse. A la suite des études menées par les inventeurs, un aspect surprenant de l'invention est que les coefficients de partage s'améliorent avec le rang de l'extraction.

Aussi lorsque l'on a à traiter un mélange réactionnel, le premier système biphasique liquide/liquide n'est pas selon l'invention. Pour systématiser et quantifier cet enseignement, il est souhaitable de préciser qu'il convient de faire en sorte que la phase aqueuse contienne en masse au moins 2/3, avantageusement au moins 3/4, de préférence au moins 8/10, plus préférentiellement 9/10, d'eau. Il est également préférable que la concentration de la phase aqueuse en base organique, le plus souvent des amines, soit au plus égale à 1 mole; avantageusement 0,5 ; de préférence 0,2 ; plus préférentiellement à 0,1 mole par kg ou par litre (en molalité ou en molarité : la différence est assez ténue).

Par carbone perhalogéné, il faut entendre un carbone d'hybridation sp³, ne portant pas d'hydrogène et comportant, outre son lien avec le chalcogène (ici le soufre), au plus 2, avantageusement au plus 1, radicaux, tous les autres atomes étant des halogènes ; lesdits radicaux sont avantageusement choisis parmi les groupes électroattracteurs (c'est-à-dire dont la constante de Hammett σₚ est supérieure à zéro mais ils sont avantageusement tels que cette constante soit au moins égale à 0,15 ; de préférence à 0,2) et ce, surtout, quand il y en a 2.

Ainsi, selon la présente invention, un carbone perhalogéné est avantageusement porteur d'au moins deux halogènes, ces deux halogènes étant avantageusement au moins partiellement et, de préférence en totalité, fluor; en d'autres termes un carbone perhalogéné est avantageusement un méthylène porteur d'un fluor et d'un autre halogène, lequel est avantageusement fluor.

On désignera ci-après une phase aqueuse par φₐ et une phase organique par φₒ.

La présente invention peut être réalisée à l'aide d'un procédé comportant plusieurs étapes d'extraction liquide-liquide au moyen d'une phase aqueuse, mais il convient qu'au moins une de ces étapes soit menée à un pH basique, c'est-à-dire que le pH après extraction reste supérieur à 7.

Au moins une des extractions liquide-liquide est menée, de manière que le pH de fin d'extraction (c'est-à-dire le pH de la phase aqueuse au sortir de l'extraction après la dernière décantation) soit au moins égal à 9, avantageusement à 10, de préférence, notamment quand il y a des proportions relativement élevées d'acides perfluoroalcanes sulfoniques à éliminer, au moins égal à 11,5. La régulation du pH peut se faire en ajoutant de la base initialement avant le contact avec la phase organique, de manière que la phase aqueuse contienne suffisamment de base pour maintenir le pH final aux valeurs précédentes. Comme cela sera rappelé ci-après, la quantité de base en équivalent à utiliser est avantageusement au moins la somme de la quantité de base nécessaire pour amener la phase aqueuse (considérée seule) au pH désiré et de la quantité en équivalent des impuretés acides salifiées par la base organique. Cette quantité est avantageusement augmentée d'une quantité allant de 0 à 20% de l'imide à récupérer, de préférence de 1 % à 10%, plus préférentiellement de 1 à 5%.

Mais on peut également ajuster le pH au cours du contact de manière à ce qu'il soit toujours et surtout en final dans la zone ci-dessus.

Pour obtenir de bons résultats, il est préférable que ledit ammonium présente un nombre de carbones au moins égal à 5, de préférence au moins égal à 6, plus préférentiellement au moins égal à 7.

Pour obtenir de bons résultats, il est préférable d'éviter que le nombre de carbones soit trop élevé, ainsi, l'on préfère que le nombre de carbones total dudit ammonium soit au plus égal à 12, avantageusement à 10. Selon la présente invention, il est préférable que l'ammonium porte au moins deux chaînes hydrocarbonées (c'est-à-dire contenant à la fois du carbone et de l'hydrogène), attaché à l'azote par un carbone secondaire d'hybridation sp³. Il est également préférable que ledit ammonium soit une amine protonée, ladite amine étant avantageusement non alcoylable.

Comme exemples d'amines non alcoylables, on peut citer les amines tertiaires comportant avantageusement comme substituant(s) au moins un radical hydrocarboné rattaché à l'azote par un carbone secondaire. Comme exemple d'amines donnant de bons résultats, on peut citer la diisopropyléthylamine, souvent désignée par son acronyme (DiPEA). Pour qu'il soit particulièrement intéressant d'utiliser le procédé selon la présente invention, il faut que la composition organique impure d'imidure d'ammonium contienne des impuretés choisies parmi les sulfonates, notamment les ions sulfonates dont le soufre est porté par un carbone perhalogéné. Il est souhaitable aussi que l'ion sulfonate dont le soufre est porté par un carbone perhalogéné ne comporte au plus que 3, avantageusement qu'au plus 2 atomes perfluorés par fonction sulfonate.

Il est également souhaitable que ledit ion sulfonate, présent comme impuretés, et présentant un soufre porté par un carbone perhalogéné, comporte au plus 8, avantageusement au plus 5, de préférence au plus 4 fluors par fonction sulfonique.

Parmi les impuretés qui sont susceptibles d'être éliminées par le procédé selon la présente invention, il convient de citer aussi les ions sulfinates qui sont souvent engendrés au cours de la réaction de synthèse des imidures ou qui peuvent être un produit de décomposition de l'imidure. Ces ions sulfinates correspondent à un anion qui présente la même formule brute que le radical sulfonyle correspondant, à la charge négative près.

Ainsi, le procédé est parfaitement adapté au traitement des compositions organiques impures d'imidure d'ammonium contenant un ion sulfinate, notamment un ion sulfinate dont le soufre est porté par un carbone perhalogéné.

Cet ion sulfinate dont le soufre est porté par un carbone perhalogéné comporte avantageusement au plus 3, de préférence au plus 2, carbones perfluorés par fonction sulfinate, fonction que l'on peut aussi désigner par l'expression "fonction sulfinique".

Il est également préférable que ledit carbone perhalogéné porté par la fonction sulfinique, ou sulfinate, comporte au plus 8, avantageusement au plus 5, de préférence au plus 4, fluors par fonction sulfinique.

Selon la présente invention, pour avoir des séparations d'excellente qualité, il est préférable que le nombre de carbones des sulfonates présents dans la composition d'imidure ou d'imide comme impuretés, soit au plus égal à 12, avantageusement à 6, de préférence à 4.

Il est préférable que la même contrainte sur le nombre total de carbones s'exerce sur les sulfinates présents comme impuretés.

Il est également préférable que le nombre de carbones perhalogénés des imidures à purifier soit au moins égal à celui des sulfinates ou des sulfonates éventuellement présents dans ladite composition.

Il est même préférable que le nombre de carbones perhalogénés desdits imidures soit supérieur à celui des sulfinates et/ou des sulfonates pour la totalité de la composition.

Il est également préférable que les carbones perhalogénés desdits imidures comportent au total au moins 2, de préférence au moins 3, fluors de plus que n'en comporte les sulfinates et/ou les sulfonates éventuellement présents dans ladite composition.

Selon la présente invention, il est préférable que ledit procédé comporte au moins deux étapes ou au moins deux séries d'étape ; une des étapes ou une des séries d'étape étant réalisée au moyen d'une phase aqueuse à pH naturel, c'est-à-dire dont le pH est celui obtenu par le simple contact avec une phase aqueuse sensiblement pure (eau de source ou eau du robinet, voire eau distillée) ou à un pH sensiblement neutre, c'est-à-dire un pH qui est régulé de manière que sa valeur en fin d'échange soit comprise dans l'intervalle fermé, c'est-à-dire comprenant les bornes de 5 à 8. Cette phase aqueuse pourra être désignée ultérieurement par φₐ₁.

L'autre étape, ou l'autre série d'étapes, d'échange liquide-liquide est réalisée par une phase aqueuse désignée ci-après par φₐ₂ dont le pH est au moins égal à 9, de préférence à 10, plus préférentiellement à 11,5, notamment quand un sulfonate est présent dans la solution d'imide ou d'imidure. En effet, bien que tous les acides présents comme impuretés soit des super acides d'acidité voisine de celle des imides, en milieu alcalin l'on observe des coefficients de partage très différents.

Lorsque l'on utilise un phase aqueuse initiale de pH compris entre 3 et 10, sans espèces tampons (c'est-à-dire dont chaque espèce présente susceptible de posséder un pouvoir tampon dans cette zone est à une concentration au plus égale à 10⁻², avantageusement à 10⁻³, il est même préférable que la somme desdites espèces réponde à cette condition), en général de l'eau de source, de l'eau distillée, permutée ou de pureté équivalente, le pH se fixe pendant la mise en contact, ce pH est appelé pH naturel. Il convient d'indiquer les conditions les plus usuelles de la mise en contact avec une phase aqueuse neutre ou à pH naturel. Ces conditions sont résumées ci-après:
- température : entre 0 et la température d'ébullition du solvant utilisé (à la pression opératoire), de préférence entre 10 et 30°C ;
- pression indifférente, de préférence à pression atmosphérique pour des raisons de facilité de mise en oeuvre ;
- rapport masse/phase aqueuse et phase organique (rapport des débits par unité de temps en cas d'opérations continues) : entre 0,1 et 10 ;
- mise en oeuvre par succession de batch mélange/décantation/soutirage ou par opération continue (colonne d'extraction ou batterie de mélangeurs décanteurs) ;
- opération par lavages méthodiques discontinu ou continu (co-courant ou contre courant).

En ce qui concerne la mise en contact avec une phase aqueuse basique, on peut indiquer que les conditions les plus courantes sont voisines, voire identiques (au pH près), comme indiqué ci-après :
- température : entre 0°C et la température d'ébullition du solvant utilisé (à la pression opératoire), de préférence entre 10 et 30°C ;
- pression (cf. ci-dessus pour la mise en contact avec la phase aqueuse neutre), de préférence à pression atmosphérique ;
- rapport entre les masses de phase aqueuse et de phase organique (rapport des débits par unité de temps en cas d'opérations continues) : entre 0,1 et 10;
- pH : compris entre 10 et 12,5 compromis entre rendement et efficacité de l'élimination des impuretés ;
- mise en oeuvre par une ou des successions d'opérations unitaires mélange/décantation/soutirage ou par opération continue (colonne d'extraction ou batterie de mélangeurs décanteurs).

Ces questions d'utilisation des technologies usuelles sont détaillées ci-après.

Les techniques d'échange liquide/liquide sont des techniques bien connues de l'homme du métier qui peuvent être des mélangeurs décanteurs en série et fonctionnant à contre-courant. Il peut également utiliser des colonnes d'échange.

Dans le cas d'une mise en oeuvre à contre-courant dans des mélangeurs décanteurs ou dans des colonnes, avec un flux aqueux jouant le rôle de la phase neutre et de la phase basique, la phase φₐ₂ correspond à la phase entrante et la phase φₐ₁ correspond à la phase sortante une fois la série des mélangeurs décanteurs parcourus par la phase aqueuse.

Il est préférable qu'il y ait un solvant, ce solvant peut être celui de la composition impure initiale, cela peut également être un solvant que l'on rajoute, cela enfin peut être un solvant que l'on a utilisé pour dissoudre l'imidure et ses impuretés en vue de l'échange.

Les solvants qui donnent les meilleurs résultats sont des solvants qui ont un doublet atome à donner, cet atome pouvant être un halogène avantageusement fluor, un chalcogène avantageusement oxygène, ou un atome de la colonne de l'azote.

Ce dernier cas n'est pas préféré car ce solvant fait double emploi avec les amines, ce qui peut alourdir le procédé. Il est également préféré que le solvant soit tel que l'atome porteur du doublet à donner ne soit pas encombré, c'est-à-dire qu'il ne porte pas plus d'un, radical carboné secondaire ou tertiaire.

Ainsi, l'on peut citer les dérivés chlorés aliphatiques comme les dérivés chlorés tels que le chlorure de méthylène ou le trichloréthylène, ces dérivés chlorés présentent avantageusement au moins un hydrogène. On peut également citer les dérivés halogénés aromatiques et l'on peut également citer les éthers non encombrés. Ainsi, l'éther éthylique donne de bons résultats, l'étherméthyle isopropylique donne également de bons résultats, en revanche, l'éther diisopropylique donne des résultats médiocres. Les carbures aromatiques tels que benzène et toluène donnent également des résultats fort médiocres.

La mise en contact avec la phase aqueuse φₐ₁ a essentiellement pour but d'éliminer les halogénures tels que fluorure, chlorure, voire bromure. La deuxième étape utilisant un pH basique vise à éliminer les sulfinates et les sulfonates. Une seule solution peut être utilisée, à condition qu'elle soit suffisamment basique.

Pour obtenir un bon taux d'élimination des acides sulfoniques, il est préférable de se placer dans un pH au moins égal à 11,5.

Lors du lavage basique, la quantité de base à utiliser est avantageusement au moins la quantité de base nécessaire pour amener la phase aqueuse (considérée seule) au pH désiré, augmentée de la quantité, en équivalent, des impuretés acides salifiées par la base organique. Cette quantité est avantageusement augmentée d'une quantité allant de 0 à 20% de l'imide à récupérer, de préférence de 1% à 10%, plus préférentiellement de 1 à 5%.

La récupération de l'imide purifié à partir de la phase organique peut être réalisée de diverses façons. Ainsi, l'on peut distiller le solvant et l'éventuelle amine excédentaire pour récupérer l'imidure d'ammonium après purification. On peut également distiller sur une base solide ou liquide plus forte que l'amine associée au dit ammonium et récupérer ainsi le triflimidure correspondant à la base.

Mais, selon une mise en oeuvre préférée de la présente invention, l'on soumet la phase organique à une solution (voire une suspension) basique c'est-à-dire dont le pH est supérieur à 12,5, avantageusement à 13.

Ainsi la solution organique débarrassée de ces impuretés peut être neutralisée par une base relativement forte, c'est-à-dire dont le pKa est au moins égal à 12,5, de préférence au moins égal à celui de la lithine. Cette neutralisation ultime permet de libérer totalement l'amine correspondant à l'ammonium, et de faire le sel de triflimidure du métal correspondant au cation de la base. Dans les étapes de lavage, il est également préférable de faire en sorte que la quantité de base ajoutée soit telle que le pH final soit inférieur à 12,5. Ainsi, il est préférable de vérifier que la dernière phase aqueuse de purification (φₐ₂ en cas de deux phases aqueuses) présente un pH qui ne soit pas trop basique et avantageusement au plus égal à 12,5.

La phase aqueuse contenant l'imidure peut être évaporée, par exemple lyophilisée.
Rappel de la signification des abréviations utilisées :
- TFSILI :: bis-trifluorométhanesulfonimidure de lithium (CF₃SO₂)₂NLi
- TFSI, NR₃ :: bis-trifluorométhanesulfonimidure de trialkylammonium
- TFSA :: trifluorométhanesulfonamide CF₃SO₂NH₂
- TFSCI :: chlorure de trifluorométhanesulfonyle CF₃SO₂Cl
- TFSIH :: bis-trifluorométhanesulfonimide
- CF₃SO₂⁻: trifluorométhanesulfinate (triflinate, TFSH)
- CF₃SO₃⁻ :: trifluorométhanesulfonate (triflate, TFSOH)
- DIPEA :: diisopropyléthylamine
- NEt₃ :: triéthylamine
- DMAP :: 4-diméthylaminopyridine

A titre d'enseignement, par l'exemple, on peut détailler la mise en oeuvre d'une séquence procédé appliqué à la synthèse de triflimidure de lithium.

Dans cette mise en oeuvre, le procédé de synthèse du bis-trifluorométhanesulfonimidure de lithium (TFSILi) comprend les étapes suivantes :
- réaction du chlorure de trifluorométhanesulfonyle (TFSCI) avec l'ammoniac pour obtenir un mélange, ici réactionnel, qui sera repris par le procédé de traitement selon l'invention ;
- élimination des co-produits et/ou des impuretés par extraction liquide-liquide selon l'invention de la masse impure ;
- récupération de l'imidure;
et optionnellement :
- élimination récupération du solvant ;
- récupération de la DIPEA par action d'une base.

### CONDITIONS OPERATOIRES PREFEREES D'ETAPES OPTIONNELLES

### Elimination du solvant

pression entre 10 hectoPa et 5 bar ;
la température est fixée par la pression;
discontinu (par lot) ou continu ;
le solvant est récupéré par condensation pour être recyclé.

### Récupération de la DIPEA par action d'une base

- par distillation azéotropique
   pression entre 10 hectoPa et 5 bar ;
   température fixée par la pression ;
   ratio molaire base/anion TFSI supérieur à 1, de préférence entre 1,01 et 1,2.
- par décantation
   ratio molaire base/anion TFSI au moins égal à 1 et avantageusement au plus égal à 3/2, de préférence 1,2 ;
   température avantageusement supérieure à 10°C pour faciliter la décantation.

Les exemples non limitatifs suivants illustrent l'invention:

### Exemplification des différentes étapes

Les exemples donnés ci-dessous pour illustrer les différentes étapes ont été choisis pour leurs qualités démonstratives. Pour des raisons évidentes à l'homme de l'art, ils n'ont pas été conduits tous sur la même échelle de quantités car certaines technologies utilisées requièrent des quantités importantes pour être menées à bien. Cela n'enlève rien à la démonstration de la faisabilité de l'enchaînement.

### Exemplification de la réaction de la composition de départ

### Exemple 1

L'appareillage utilisé comprend:
- un réacteur de 1 litre double enveloppé, équipé d'une agitation centrale;
- un bain chaud/froid permettant de maintenir la masse réactionnelle à -30°C si besoin ;
- une alimentation d'ammoniac gaz par plongeant à partir d'une bouteille d'ammoniac sous pression de 1 litre ;
- un piège à carboglace en série sur un jeu de soupapes N₂ permettant le maintien du réacteur à une pression proche de la pression atmosphérique du laboratoire ;
- une injection de CF₃SO₂Cl à débit contrôlé via un pousse seringue.
L'ensemble de l'appareillage est placé dans une sorbonne ventilée.
Dans le réacteur de 1 litre, on charge du dichlorométhane (490 g), la DIPEA (221 g) et la DMAP (3,5 g).
La quantité souhaitée d'ammoniac (9,7 g) est chargée par absorption à -15°C dans le pied dichlorométhanique à partir de la bouteille par l'intermédiaire du plongeur. La quantité exacte chargée est déterminée par pesée et/ou mesure de débit. Le condenseur refroidi à la carboglace assure qu'il n'y a pas de perte de l'ammoniac hors du réacteur. La solution de CF₃SO₂Cl à 50% dans le dichlorométhane (383,4 g) est ensuite injectée via un pousse-seringue en maintenant la température de la masse réactionnelle entre -10 et -5°C : durée de coulée 3 heures. On laisse ensuite la température remonter à l'ambiante (1 heure). La masse réactionnelle est alors lavée par environ 150 g d'eau pour avoir deux phases homogènes facilement analysables. On récupère environ 1002,7 g de phase organique et 240,3 g de phase aqueuse.
L'analyse de la composition des phases organique et dichlorométhanique par chromatographie ionique donne les résultats suivants :

| Phase | Organique | Aqueuse | Coef. de partage φₒ/φₐ |
|---|---|---|---|
| poids | % | % | |
| Cl- | 1,9 | 8,5 | 0,23 |
| CF₃SO₂⁻ | 0,33 | 0,07 | 4,7 |
| CF₃SO₃⁻ | 1,2 | 0,2 | 5,7 |
| (CF₃SO)₂N- | 14,8 | nd | |

Ces coefficients de partage ne sont guère favorables à la purification.

### Exemple 2

On répète l'exemple 1, à ceci près qu'on charge la totalité du dichlorométhane (680 g) dans le réacteur en début d'opération. On charge ensuite 268,6 g de DIPEA, 4,2 g de DMAP et 12,0 g d'ammoniac. Le CF₃SO₂Cl (233,2 g) est alors injecté dans le milieu réactionnel par l'intermédiaire d'un pousse-seringue. La durée d'injection est de 2h30 mn pendant lesquelles la masse réactionnelle est maintenue à -20°C. On laisse ensuite la température remonter à l'ambiante (1 heure). La masse réactionnelle est alors lavée par environ 300 g d'eau pour avoir deux phases homogènes facilement analysables. On récupère environ 1040 g de phase organique et 445 g de phase aqueuse.

| % en masse | Masse des phases | F⁻ | Cl⁻ | CF₃SO₂⁻ | CF₃SO₂NH₂ | CF₃SO₃⁻ | (CF₃SO)₂N⁻ |
|---|---|---|---|---|---|---|---|
| φₒ | 1040 g | 0 | 1,5 | 0,2 | 0,05 | 0,6 | 18 |
| φₐ | 445 g | 0 | 7,2 | 0,05 | 0,01 | 0,08 | |
| Coef de partage φₒ/φₐ | | | 0,2 | 4 | 5 | 7,5 | |

Les coefficients de partage sont ici très défavorables à la purification.

### Exemplification des lavages par extraction liquide-liquide

### Exemple 3 - extraction à pH neutre ou naturel

Cette exemple illustre la manière d'effectuer les lavages aqueux pour éliminer les ions chlorures.

On répète l'exemple 1, à ceci prés que l'ammoniac est chargé à une température plus froide (-20°C), ce qui permet de charger une quantité d'ammoniac plus importante. La charge de DIPEA, TFSCI, DMAP est modifiée en proportion. La quantité totale de dichlorométhane chargée est inchangée. Après réaction, la masse réactionnelle est lavée par 300 g d'eau. Après décantation, la phase aqueuse est éliminée du réacteur et on rajoute 300 g d'eau. On agite l'ensemble pendant ¼ d'heure puis on laisse décanter également pendant ¼ d'heure. Les phases organique et aqueuse sont analysées par chromatographie ionique puis on élimine de nouveau la phase aqueuse. On répète cette opération quatre fois. L'évolution de la composition des différentes phases est illustrée dans les tableaux ci-après.

On observe que les ions chlorures gênants pour la suite du procédé et pour la qualité du produit final sont éliminés de la phase organique avec des pertes minimes de l'anion TFSI à valoriser.

| Phase | Espèces chimiques | | | | | |
|---|---|---|---|---|---|---|
| | F⁻ | Cl⁻ | CF₃SO₂⁻ | CF₃SO₂NH⁻ | CF₃SO₃⁻ | TFSI⁻ |
| Phase organique 1^{er} lavage | 0,001 | 2 | 0,2 | 0,05 | 0,6 | 24 |
| Phase aqueuse 1^{er} lavage | 0,001 | 7,4 | 0,04 | 0,05 | 0,08 | 0,05 |
| Phase organique 2^{ème} lavage | 0,001 | 0,7 | 0,2 | 0,06 | 0,6 | 26,5 |
| Phase aqueuse 2^{ème} lavage | 0,001 | 3,1 | 0,06 | 0,03 | 0,09 | 0,18 |
| Phase organique 3^{ème} lavage | 0,001 | 0,2 | 0,15 | 0,03 | 0,6 | 27,5 |
| Phase aqueuse 3^{ème} lavage | 0,001 | 1,2 | 0,08 | 0,015 | 0,1 | 0,06 |
| Phase organique 4^{ème} lavage | 0,001 | 0,03 | 0,1 | 0,05 | 0,5 | 27,8 |
| Phase aqueuse 4^{ème} lavage | 0,001 | 0,3 | 0,1 | 0,017 | 0,2 | 0,1 |

| | Coefficient de partage φₒ/φₐ | | | | | | Rapport entre le coefficient de partage du TFSI et celui de l'espèce chimique considérée | | | | | |
|---|---|---|---|---|---|---|---|---|---|---|---|---|
| | F⁻ | Cl⁻ | CF₃SO₂⁻ | CF₃SO₂NH⁻ | CF₃SO₃⁻ | TFSI⁻ | F⁻ | Cl⁻ | CF₃SO₂⁻ | CF₃SO₂NH⁻ | CF₃SO₃⁻ | TFSI⁻ |
| 1^{er} lavage | 1 | 0,270 | 5 | 1 | 7,5 | 480 | 480 | 1776 | 96 | 480 | 64 | 1 |
| 2^{ème} lavage | 1 | 0,226 | 3,333 | 2 | 6,667 | 147,2 | 147,22 | 651,9 | 44,17 | 73,61 | 22,08 | 1 |
| 3^{ème} lavage | 1 | 0,167 | 1,875 | 2 | 6 | 458,3 | 458,33 | 2750 | 244,44 | 229,17 | 76,39 | 1 |
| 4^{ème} lavage | 1 | 0,1 | 1 | 2,941 | 2,5 | 278 | 278 | 2780 | 278 | 94,52 | 111,2 | 1 |

### Exemple 4

Cet exemple illustre la manière de laver la masse réactionnelle à pH basique pour éliminer les impuretés comme le trifluorométhanesulfonate ou le trifluorométhanesulfinate.

Dans le réacteur de 1 litre décrit précédemment mais équipé d'une sonde pH, on place une masse réactionnelle de masse 792 g, obtenue comme dans l'exemple 3, c'est à dire préalablement lavée pour être débarrassée des ions chlorures et après avoir évaporé une partie du solvant dichlorométhane (composition phase organique 1), on ajoute alors sous agitation 300 g d'eau et 26 ml de soude 2N pour obtenir un pH de 10,8. On laisse décanter ¼ d'heure et on retire la phase aqueuse. On ajoute à nouveau 300 g d'eau et on ajuste sous agitation le pH à 10,8 par cette fois 8,5 ml de soude 2N. Après décantation on retire la phase aqueuse.

Le poids et la composition des différentes phases sont donnés dans les tableaux ci-après.

On observe que dans la phase organique 3, la teneur en anion trifluorométhane-sulfonique et en anion trifluorométhanesulfinique est respectivement inférieure à 0,06% et 0,01%. Ceci démontre qu'après trois lavages basiques on obtient une solution dichlorométhanique du complexe TFSIH/DIPEA pratiquement pur.

| | poids | F⁻ | Cl⁻ | CF₃SO₂⁻ | CF₃SO₂NH⁻ | CF₃SO₃⁻ | TFSI⁻ |
|---|---|---|---|---|---|---|---|
| Phase organique 1 | 792 | <10⁻³ | 0,04 | 0,1 | 0,1 | 0,6 | 30,7 |
| Phase organique 2 | 767 | <10⁻³ | 0,006 | 0,01 | 0,01 | 0,16 | 30,9 |
| Phase aqueuse 2 | 342 | <10⁻³ | 0,07 | 0,2 | 0,1 | 1 | 1,4 |
| Phase organique 3 | 746 | <10⁻³ | 0,004 | 0,01 | 0,01 | 0,06 | 30,7 |
| Phase aqueuse 3 | 321 | <10⁻³ | <10⁻³ | 0,02 | 0,01 | 0,2 | 1,1 |

| | Coefficient de partage φₒ/φₐ | | | | | | | Rapport entre le coefficient de partage du TFSI et celui de l'espèce chimique considérée | | | | | |
|---|---|---|---|---|---|---|---|---|---|---|---|---|---|
| | poids | F⁻ | Cl⁻ | CF₃SO₂⁻ | CF₃SO₂NH⁻ | CF₃SO₃⁻ | TFSI⁻ | F⁻ | Cl⁻ | CF₃SO₂⁻ | CF₃SO₂NH⁻ | CF₃SO₃⁻ | TFSI⁻ |
| 1^{er} lavage | 2,24 | 1,00 0,09 | | 0,05 | 0,10 | 0,16 | 22,07 | 22,07 | 257,50 | 441,43 | 220,71 | 137,95 | 1,00 |
| 2^{ème} lavage | 2,32 | 1,00 | n.s. | 0,50 | 1,00 | 0,30 | 27,91 | 27,91 | n.s. | 55,82 | 27,91 | 93,03 | 1,00 |

## Revendications

1. Procédé de traitement d'une composition organique impure d'imidure d'ammonium, imidure dont l'un des substituants est un sulfonyle porté par un carbone perhalogéné, de préférence perfluoré, **caractérisé par le fait que** l'on soumet ladite composition à une étape d'extraction liquide-liquide au moyen d'une phase aqueuse Φₐ présentant un pH ajusté à une valeur au moins égale 9 et comportant comme impureté au moins l'une des espèces chimiques choisies parmi les halogénures, les sulfonates et les sulfinates.

2. Procédé selon la revendication 1, **caractérisé par le fait que** la phase aqueuse Φₐ présente un pH ajusté à une valeur au moins égale à 10, de préférence à 11,5, quand un acide perfluorosulfonique est présent.

3. Procédé selon les revendications 1 et 2, **caractérisé par le fait que** ledit ammonium présente un nombre de carbone au moins égal à 5 et de préférence au plus à 12.

4. Procédé selon les revendications 1 à 3, **caractérisé par le fait que** l'ammonium porte au moins 2 chaînes hydrocarbonées (c'est-à-dire contenant à la fois du carbone et de l'hydrogène) attachées à l'azote par un carbone secondaire d'hybridation sp³.

5. Procédé selon les revendications 1 à 4, **caractérisé par le fait que** ledit ammonium est une amine protonée.

6. Procédé selon les revendications 1 à 5, **caractérisé par le fait que** ledit ammonium est une amine non alcoylable protonée.

7. Procédé selon les revendications 1 à 6, **caractérisé par le fait que** ledit ammonium est une amine tertiaire protonée.

8. Procédé selon les revendications 1 à 7, **caractérisé par le fait que** ledit ammonium est une amine tertiaire non quatemisable protonée.

9. Procédé selon les revendications 1 à 8, **caractérisé par le fait que** ladite composition organique impure d'imidure d'ammonium contient un ion sulfonate dont le soufre est porté par un carbone perhalogéné.

10. Procédé selon les revendications 1 à 9, **caractérisé par le fait que** ladite composition organique impure d'imidure d'ammonium contient un ion sulfonate dont le soufre est porté par un carbone perhalogéné et qui comporte au plus trois, de préférence au plus deux, carbones perfluorés par fonction sulfonate.

11. Procédé selon les revendications 1 à 10, **caractérisé par le fait que** ladite composition organique impure d'imidure d'ammonium contient un ion sulfonate dont le soufre est porté par un carbone perhalogéné et qui comporte au plus 8, de préférence au plus 5, et encore plus préférentiellement au plus 4, fluors par fonction sulfonique.

12. Procédé selon les revendications 1 à 11, **caractérisé par le fait que** ladite composition organique impure d'imidure d'ammonium contient un ion sulfinate dont le soufre est porté par un carbone perhalogéné.

13. Procédé selon les revendications 1 à 12, **caractérisé par le fait que** ladite composition organique impure d'imidure d'ammonium contient un ion sulfinate dont le soufre est porté par un carbone perhalogéné et qui comporte au plus trois, de préférence au plus deux, carbones perfluorés par fonction sulfinate.

14. Procédé selon les revendications 1 à 13, **caractérisé par le fait que** ladite composition organique impure d'imidure d'ammonium contient un ion sulfinate dont le soufre est porté par un carbone perhalogéné et qui comporte au plus 8, de préférence au plus 5, de préférence au plus 4, fluors par fonction sulfinique.

15. Procédé selon les revendications 1 à 14, **caractérisé par le fait que** ledit imidure est porteur de deux fonctions sulfonyles dont l'une au moins est portée par un carbone perhalogéné, de préférence perfluoré.

16. Procédé selon les revendications 1 à 15, **caractérisé par le fait que** ledit imidure est porteur de deux fonctions sulfonyles, toutes deux portées par un carbone perhalogéné, de préférence perfluoré.

17. Procédé selon les revendications 1 à 16, **caractérisé par le fait que** le nombre de carbones des sulfonates est au plus égal à 12, de préférence à 6, plus préférentiellement à 4.

18. Procédé selon les revendications 1 à 17, **caractérisé par le fait que** le nombre de carbones des sulfinates est au plus égal à 12, de préférence à 6, plus préférentiellement à 4.

19. Procédé selon les revendications 1 à 18, **caractérisé par le fait que** le nombre de carbones perhalogénés desdits imidures est au moins égal à celui des sulfinates et/ou des sulfonates éventuellement présents dans ladite composition.

20. Procédé selon les revendications 1 à 19, **caractérisé par le fait que** le nombre de carbones perhalogénés desdits imidures est supérieur à celui des sulfinates et/ou des sulfonates éventuellement présents dans ladite composition.

21. Procédé selon les revendications 1 à 20, **caractérisé par le fait que** le nombre de carbones perhalogénés desdits imidures comporte au moins 2, de préférence au moins 3, fluors de plus que n'en comportent les sulfinates et/ou les sulfonates éventuellement présents dans ladite composition.

22. Procédé selon les revendications 1 à 21, **caractérisé par le fait que** ledit procédé comporte au moins deux étapes d'échange liquide-liquide dont l'une a) est réalisée par une phase aqueuse Φₐ₁ à pH naturel ou neutre (pH compris entre 5 et 8) et dont l'autre l'est par une phase aqueuse Φₐ₂ dont le pH est au moins égal à 9, de préférence à 10.

## Claims

1. A process for treating an impure organic ammonium imide composition, one of the substituents of which imide is a sulphonyl carried by a perhalogenated carbon, preferably perfluorinated, **characterized in that** said composition undergoes a liquid-liquid extraction step using an aqueous phase Φₐ with a pH adjusted to a value of at least 9 and including at least one chemical species selected from halides, sulphonates and sulphinates as an impurity.

2. A process according to claim 1, **characterized in that** the pH of the aqueous phase Φₐ is adjusted to a value of at least 10, preferably at least 11.5, when a perfluorosulphonic acid is present.

3. A process according to claim 1 or claim 2, **characterized in that** the number of carbons in said ammonium is at least 5 and preferably at most 12.

4. A process according to claims 1 to 3, **characterized in that** the ammonium carries at least 2 hydrocarbon chains (i.e. containing both carbon and hydrogen) attached to the nitrogen via a secondary carbon with sp³ hybridization.

5. A process according to claims 1 to 4, **characterized in that** said ammonium is a protonated amine.

6. A process according to claims 1 to 5, **characterized in that** said ammonium is a protonated non alkoylable amine.

7. A process according to claims 1 to 6, **characterized in that** said ammonium is a protonated tertiary amine.

8. A process according to claims 1 to 7, **characterized in that** said ammonium is a protonated non quaternizable tertiary amine.

9. A process according to claims 1 to 8, **characterized in that** said impure organic ammonium imide composition contains a sulponate ion the sulphur of which is carried by a perhaogenated carbon.

10. A process according to claims 1 to 9, **characterized in that** said impure organic ammonium imide composition contains a sulphonate ion the sulphur of which is carried by a perhalogenated carbon and which includes at most three, preferably at most two perfluorinated carbons per sulphonate function.

11. A process according to claims 1 to 10, **characterized in that** said impure organic ammonium imide composition contains a sulphonate ion the sulphur of which is carried by a perhalogenated carbon and which comprises at most 8, preferably at most 5 and more preferably at most 4 fluorines per sulphonic function.

12. A process according to claims 1 to 11, **characterized in that** said impure organic ammonium imide composition contains a sulphinate ion the sulphur of which is carried by a perhalogenated carbon.

13. A process according to claims 1 to 12, **characterized in that** said impure organic ammonium imide composition contains a sulphinate ion the sulphur of which is carried by a perhalogenated carbon and which comprises at most three, preferably at most two perfluorinated carbons per sulphinate function.

14. A process according to claims 1 to 13, **characterized in that** said impure organic ammonium imide composition contains a sulphinate ion the sulphur of which is carried by a perhalogenated carbon and which comprises at most 8, preferably at most 5, preferably at most 4 fluorines per sulphinic function.

15. A process according to claims 1 to 14, **characterized in that** said imide carries two sulphonyl functions at least one of which is carried by a perhalogenated carbon, preferably perfluorinated.

16. A process according to claims 1 to 14, **characterized in that** said imide carries two sulphonyl functions both of which being carried by a perhalogenated, preferably perfluorinated carbon.

17. A process according to claims 1 to 16, **characterized in that** the number of carbons in the sulphonates is at most 12, preferably at most 6, more preferably at most 4.

18. A process according to claims 1 to 17, **characterized in that** the number of carbons in the sulphinates is at most 12, preferably at most 6, more preferably at most 4.

19. A process according to claims 1 to 18, **characterized in that** the number of perhalogenated carbons in said imides is at least equal to that of the sulphinates and/or sulphonates optionally present in said composition.

20. A process according to claims 1 to 19, **characterized in that** the number of perhalogenated carbons in said imides is greater than that of the sulphinates and/or sulphonates optionally present in said composition.

21. A process according to claims 1 to 20, **characterized in that** the number of perhalogenated carbons in said imides comprises at least 2, preferably at least 3 fluorines more than comprised in the sulphinates and/or sulponates optionally present in said composition.

22. A process according to claims 1 to 21, **characterized in that** said process comprises at least two liquid-liquid exchange steps wherein one a) is carried out by an aqueous phase Φₐ₁ at a natural or neutral pH (a pH in the range 5 to 8) and the other is carried out using an aqueous phase Φₐ₂ the pH of which is at least 9, preferably at least 10.

## Patentansprüche

1. Verfahren zur Behandlung einer mit Ammoniumimid verunreinigten organischen Zusammensetzung, wobei einer der Substituenten des Imids ein Sulfonyl ist, getragen durch einen perhalogenierten, vorzugsweise perfluorierten Kohlenstoff, **dadurch gekennzeichnet, dass** man die genannte Zusammensetzung einer Stufe zur Flüssig-Flüssig-Extraktion mit Hilfe einer wässerigen Phase Φₐ unterzieht, die einen pH-Wert aufweist, der auf mindestens gleich 9 eingestellt ist und die als Verunreinigung mindestens eine der chemischen Arten umfasst, die ausgewählt sind unter den Halogeniden, den Sulfonaten und den Sulfinaten.

2. Verfahren nach Anspruch 1, **dadurch gekennzeichnet, dass** die wässerige Phase Φₐ einen pH-Wert aufweist, der auf mindestens gleich 10, vorzugsweise 11,5 eingestellt ist, wenn eine Perfluorsulfonsäure anwesend ist.

3. Verfahren nach Anspruch 1 und 2, **dadurch gekennzeichnet, dass** das genannte Ammonium eine Anzahl von Kohlenstoffen von mindestens gleich 5 und vorzugsweise höchstens 12 aufweist.

4. Verfahren nach Anspruch 1 bis 3, **dadurch gekennzeichnet, dass** das Ammonium mindestens zwei Kohlenwasserstoff-Ketten (das heißt, sowohl Kohlenstoff als auch Wasserstoff enthaltend) trägt, gebunden an den Stickstoff durch einen sekundären Kohlenstoff der Hybridisierung sp³.

5. Verfahren nach Anspruch 1 bis 4, **dadurch gekennzeichnet, dass** das genannte Ammonium ein protonisiertes Amin ist.

6. Verfahren nach Anspruch 1 bis 5, **dadurch gekennzeichnet, dass** das genannte Ammonium ein protonisiertes, nicht alkylierbares Amin ist.

7. Verfahren nach Anspruch 1 bis 6, **dadurch gekennzeichnet, dass** das genannte Ammonium ein protonisiertes, tertiäres Amin ist.

8. Verfahren nach Anspruch 1 bis 7, **dadurch gekennzeichnet, dass** das genannte Ammonium ein protonisiertes, nicht quaternisierbares tertiäres Amin ist.

9. Verfahren nach Anspruch 1 bis 8, **dadurch gekennzeichnet, dass** die genannte organische Zusammensetzung, die mit Ammoniumimid verunreinigt ist, ein Sulfonat-Ion enthält, bei dem der Schwefel durch einen perhalogenierten Kohlenstoff getragen wird.

10. Verfahren nach Anspruch 1 bis 9, **dadurch gekennzeichnet, dass** die genannte organische Zusammensetzung, die mit Ammoniumimid verunreinigt ist, ein Sulfonat-Ion enthält, bei dem der Schwefel durch einen perhalogenierten Kohlenstoff getragen wird, und das höchstens drei, vorzugsweise höchstens zwei perfluorierte Kohlenstoffe pro Sulfonat-Funktion umfasst.

11. Verfahren nach Anspruch 1 bis 10, **dadurch gekennzeichnet, dass** die genannte organische Zusammensetzung, die mit Ammoniumimid verunreinigt ist, ein Sulfonat-Ion enthält, bei dem der Schwefel durch einen perhalogenierten Kohlenstoff getragen wird, und das höchstens acht, vorzugsweise höchstens fünf und noch bevorzugter höchstens vier Fluor pro Sulfonat-Funktion umfasst.

12. Verfahren nach Anspruch 1 bis 11, **dadurch gekennzeichnet, dass** die genannte organische Zusammensetzung, die mit Ammoniumimid verunreinigt ist, ein Sulfinat-Ion enthält, bei dem der Schwefel durch einen perhalogenierten Kohlenstoff getragen wird.

13. Verfahren nach Anspruch 1 bis 12, **dadurch gekennzeichnet, dass** die genannte organische Zusammensetzung, die mit Ammoniumimid verunreinigt ist, ein Sulfinat-Ion enthält, bei dem der Schwefel durch einen perhalogenierten Kohlenstoff getragen wird, und das höchstens drei, vorzugsweise höchstens zwei perfluorierte Kohlenstoffe pro Sulfinat-Funktion umfasst.

14. Verfahren nach Anspruch 1 bis 13, **dadurch gekennzeichnet, dass** die genannte organische Zusammensetzung, die mit Ammoniumimid verunreinigt ist, ein Sulfinat-Ion enthält, bei dem der Schwefel durch einen perhalogenierten Kohlenstoff getragen wird, und das höchstens acht, vorzugsweise höchstens fünf und noch bevorzugter höchstens vier Fluor pro Sulfinat-Funktion umfasst.

15. Verfahren nach Anspruch 1 bis 14, **dadurch gekennzeichnet, dass** das genannte Imid Träger von zwei Sulfonyl-Funktionen ist, von denen mindestens eine durch einen perhalogenierten, vorzugsweise perfluorierten Kohlenstoff getragen wird.

16. Verfahren nach Anspruch 1 bis 15, **dadurch gekennzeichnet, dass** das genannte Imid Träger von zwei Sulfonyl-Funktionen ist, die alle beide durch einen perhalogenierten, vorzugsweise perfluorierten Kohlenstoff getragen werden.

17. Verfahren nach Anspruch 1 bis 16, **dadurch gekennzeichnet, dass** die Anzahl der Kohlenstoffe der Sulfonate höchstens gleich 12, vorzugsweise 6 und noch mehr bevorzugt 4 beträgt.

18. Verfahren nach Anspruch 1 bis 17, **dadurch gekennzeichnet, dass** die Anzahl der Kohlenstoffe der Sulfinate höchstens gleich 12, vorzugsweise 6 und noch mehr bevorzugt 4 beträgt. ,

19. Verfahren nach Anspruch 1 bis 18, **dadurch gekennzeichnet, dass** die Anzahl der perhalogenierten Kohlenstoffe der genannten Imide mindestens gleich der Anzahl der Sulfinate und/oder Sulfonate ist, die gegebenenfalls in der genannten Zusammensetzung anwesend sind.

20. Verfahren nach Anspruch 1 bis 19, **dadurch gekennzeichnet, dass** die Anzahl der perhalogenierten Kohlenstoffe der genannten Imide höher ist als die Anzahl der Sulfinate und/oder Sulfonate, die gegebenenfalls in der genannten Zusammensetzung anwesend sind.

21. Verfahren nach Anspruch 1 bis 20, **dadurch gekennzeichnet, dass** die Anzahl der perhalogenierten Kohlenstoffe der genannten Imide mindestens 2, vorzugsweise mindestens 3 Fluor mehr als die Anzahl der Sulfinate und/oder Sulfonate umfasst, die gegebenenfalls in der genannten Zusammensetzung anwesend sind.

22. Verfahren nach Anspruch 1 bis 21, **dadurch gekennzeichnet, dass** das genannte Verfahren mindestens zwei Stufen des Flüssig-Flüssig-Austausches umfasst, bei dem eine a) durch eine wässerige Phase Φₐ₁ bei natürlichem oder neutralem pH-Wert (pH zwischen 5 und 8) realisiert wird und die andere eine wässerige Phase Φₐ₂ ist, deren pH-Wert mindestens gleich 9, vorzugsweise 10 beträgt.
